Europäisches Patentamt

European Patent Office

Office européen des brevets

(19)

(11) Veröffentlichungsnummer : **0 338 981 B1**

(12) **EUROPÄISCHE PATENTSCHRIFT**

(45) Veröffentlichungstag der Patentschrift :
**12.08.92 Patentblatt 92/33**

(51) Int. Cl.⁵ : **A61F 2/30**

(21) Anmeldenummer : **89810262.9**

(22) Anmeldetag : **06.04.89**

(54) Markraumsperre.

(30) Priorität : **22.04.88 CH 1518/88**

(43) Veröffentlichungstag der Anmeldung :
**25.10.89 Patentblatt 89/43**

(45) Bekanntmachung des Hinweises auf die
Patenterteilung :
**12.08.92 Patentblatt 92/33**

(84) Benannte Vertragsstaaten :
**AT BE CH DE FR GB IT LI LU NL SE**

(56) Entgegenhaltungen :
**EP-A- 0 018 496
DE-A- 2 910 627
DE-A- 3 445 709
FR-A- 2 570 606
US-A- 4 186 448**

(73) Patentinhaber : **Mathys AG Bettlach
Güterstrasse 5
CH-2544 Bettlach (CH)**

(72) Erfinder : **Weigum, Hans
Hofergässli 4
CH-2544 Bettlach (CH)**
Erfinder : **Mathys, Robert jr.
Güterstrasse 5
CH-2544 Bettlach (CH)**

(74) Vertreter : **Eder, Carl E. et al
Patentanwaltsbüro EDER AG
Lindenhofstrasse 40
CH-4052 Basel (CH)**

EP 0 338 981 B1

**Beschreibung**

Hüftgelenkprothesen werden in der Markhöhle des Oberschenkelknochens meist mittels eines aus Polymethylmetacrylat bestehenden Knochenzementes verankert. Dieser wird in pastöser Form in die Markhöhle eingebracht. Dann wird in die noch plastische Masse der Prothesenschaft eingedrückt, wonach der Zement ganz aushärten kann. Es ist dabei darauf zu achten, dass der Knochenzement möglichst nur so tief aber nicht tiefer in die Markhöhle eingebracht wird, dass er nach dem Eindrücken des Prothesenschafts den ganzen Raum zwischen Knochen und Prothesenschaft sicher füllt. Der beim Eindrücken des Prothesenschaftes entstehende Druck soll eine Verdichtung des Knochenzements bewirken, damit die Hohlraumbildung durch Lufteinschlüsse gering gehalten und die Anschmiegung des Zement an die Konturen der Markhöhle verbessert wird, was schwieriger ist, wenn der noch plastische Zement beim Eindrücken des Prothesenschaftes in die freie Markhöhle entweichen kann.

Um dieses unerwünschte Ausweichen des Knochenzements zu verhindern, sind bereits verschiedene Markraumsperren bekannt und zwar solche aus körpereigenen Materalien (also aus Knochenspongiosa) wie auch solche aus synthetischem Material. Eine derartige Markraumsperre muss aus körperverträglichem Material bestehen. Ausserdem soll sie deformierbar sein, da der Pfropfen den Markraumkanal unter Umständen jenseits seiner engsten Stelle und üblicherweise an einer Stelle mit von der idealen Kreisfläche abweichendem Querschnitt abschliessen soll. Des weiteren ist es zweckmässig, wenn der Werkstoff nicht nur körperverträglich ist sondern vom Körper möglichst rasch resorbiert werden kann, da er nach dem Erstarren des Zements, also schon nach 15 Min., keine funktionale Wirkung mehr hat.

Markraumsperren aus Knochenspongiosa weisen eine gute Körperverträglichkeit auf, sie werden rasch resorbiert bzw. umgebaut. Der Nachteil derartiger Sperren besteht darin, dass sie kaum deformierbar sind, dass sie ein gegenüber Markraumsperren aus synthetischem Material erhöhtes Infektions-Risiko aufweisen, weil sie ja nur beschränkt desinfizierbar sind, und dass die Operationsdauer verlängert wird, wenn sie aus Knochenmaterial angefertigt werden, das bei der gleichen Operation anfällt, also beispielsweise aus der abgetrennten Gelenkkugel.

Die bekannten Markraumsperren aus syntethischem Material, z. B. aus Dextrose, können zwar gut sterilisiert werden und werden vom Körper rasch resorbiert, haben aber wegen ihrer mangelnden Deformierbarkeit nur eine beschränkte Abschlusswirkung. An sich hat dieses Material zwar die Eigenschaft, dass es aufquellen kann. Um diese Eigenschaft aber auszunützen ist die zur Verfügung stehende Zeit zu kurz.

Eine Markraumsperre gemäß dem Oberbegriff des Anspruchs 1 ist aus der DE-A-34 45 709 bekannt.

Die erfindungsgemässe Markraumsperre besitzt nun alle Vorteile der bekannten Markraumsperren, ohne jedoch ihre Nachteile aufweisen zu müssen. Sie ist dadurch gekennzeichnet, dass der Pfropfen porös ist und aus regellos gepackten, gekräuselten Fadenstücken aus einem resorbierbaren, polymeren Werkstoff besteht. Die vorgenannten Fadenstücke sind vorteilhafterweise monofil. Besonders zweckmässig ist es, wenn der Pfropfen kegelstumpfförmig gepresst und derart formfixiert ist, dass er elastisch deformierbar ist. Als resorbierbares Material lässt sich zweckmässig ein resorbierbarer polymerer Werkstoff, wie z. B. ein Poly-Lactic-Acid verwenden.

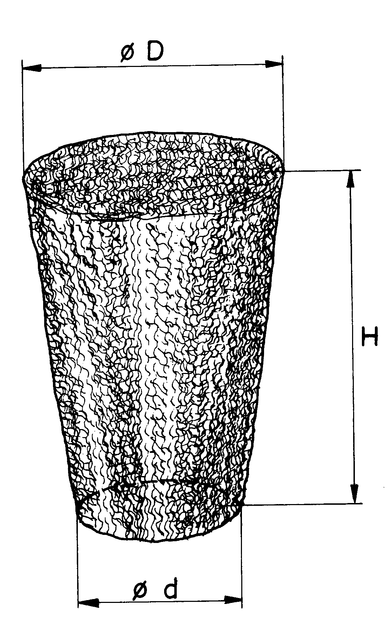

Wegen der durch die Konstruktion gewährleisteten elastischen Deformierbarkeit passt er sich der Markraumhöhle in bezug auf Dimension und Form ohne weiteres sehr gut an, so dass sich Sperren einheitlicher Grösse für die meisten Anwendungsfälle verwenden lassen. Diese Konstruktion hat zudem eine selektive Sperrwirkung zur Folge, so dass gegenüber viskosen Substanzen, wie dem Knochenzement, der Pfropfen undurchlässig ist, während er für Luft, Spülflüssigkeit und Blut durchlässig ist, sodass diese Medien durch den Knochenzement verdrängt werden können, wodurch sich weniger Einschlüsse im Knochenzement und an der Grenzfläche zwischen Knochenzement und Knochen bilden können, was die Qualität des ausgehärteten Zements wesentlich erhöht. Die ebenfalls durch die geometrische Konstruktion bewirkte grosse spezifische Oberfläche ist eine gute Voraussetzung für eine rasche Resorption. Die einzige Figur der beiliegenden Zeichnung zeigt ein Ausführungsbeispiel einer erfindungsgemässen Markraumsperre, die als kegelstumpfförmiger Pfropfen ausgebildet ist. Er hat etwa folgende Abmessungen: Durchmesser an der Basis D = ca. 15 mm, Durchmesser an der Spitze d = ca. 10 mm, Höhe H = ca. 20 mm.

**Patentansprüche**

1. Resorbierbare, als Pfropfen ausgebildete Markraumsperre, dadurch gekennzeichnet, dass der Pfropfen porös ist und aus regellos gepackten, gekräuselten Fadenstücken aus einem resorbierbaren, polymeren Werkstoff besteht.

2. Markraumsperre nach Anspruch 1, dadurch gekennzeichnet, dass der Pfropfen kegelstumpfförmig gepresst und derart formfixiert ist, dass er elastisch deformierbar ist.

## Claims

1. Resorbable marrow cavity closure constructed as plug, characterised thereby that the plug is porous and consists of randomly packed, crinkled pieces of thread of a resorbable, polymer material.

2. Marrow cavity closure according to claim 1, characterised thereby that the plug is pressed to be frustoconically-shaped and is fixed in shape in such a manner that it is elastically deformable.

## Revendications

1. Bouchon résorbable pour le canal médullaire, conçu sous forme de tampon, caractérisé en ce que le tampon est poreux et se compose de morceaux de fibres ondulées comprimées de manière irrégulière et constituées d'une matière polymère résorbable.

2. Bouchon pour le canal médullaire selon la revendication 1, caractérisé en ce que le tampon est comprimé en forme de cône tronqué, sa conformation étant telle qu'il est élastiquement déformable.